# EUROPÄISCHE PATENTSCHRIFT

(11) **EP 3 239 873 B1**
(45) Veröffentlichungstag und Bekanntmachung des Hinweises auf die Patenterteilung: **26.06.2019**
(21) Anmeldenummer: 17170249.1
(22) Anmeldetag: 09.05.2017
(51) Int. Cl.: G16H 40/60

(54) **VERFAHREN UND GERÄTESYSTEM ZUM MANAGEMENT DER FUNKTIONSAUSÜBUNG VON MEDIZINTECHNISCHEN GERÄTEN**
METHOD AND DEVICE SYSTEM FOR MANAGING THE FUNCTIONALITY OF MEDICAL DEVICES
PROCÉDÉ ET SYSTÈME D'APPAREIL DESTINÉS À LA GESTION DU FONCTIONNEMENT D'APPAREILS TECHNIQUES MÉDICAUX

(43) Veröffentlichungstag der Anmeldung: 01.11.2017
(60) Teilanmeldung: 19163749.5
(73) Patentinhaber: Siemens Healthcare GmbH, 91052 Erlangen (DE)
(72) Erfinder: Jörger, Clemens, 91301 Forchheim (DE)

(56) Entgegenhaltungen:
- EP-A2- 1 091 522
- US-A1- 2006 242 148
- US-A1- 2010 138 523
- US-A1- 2013 198 569
- US-A1- 2017 124 271
- US-B2- 8 520 017

## Beschreibung

Die Erfindung betrifft ein Verfahren zum Management der Funktionsausübung von medizintechnischen Geräten. Die Erfindung betrifft des Weiteren ein für ein solches Management ausgestattetes medizintechnisches Gerät und ein Gerätesystem umfassend solche medizintechnischen Geräte.

Heutzutage sind bei medizintechnischen Geräten, z. B. bei bildgebenden Systemen wie Computertomographen oder Röntgengeräten, Ultraschallgeräten, digitalen Röntgendetektoren etc., durch den Benutzer mannigfaltige Eingaben und Einstellungen vorzunehmen, die dem Zugriff bzw. der Authentifizierung, der individuellen Konfigurierung oder der Kalibrierung dienen. Diese Einstellungen können in der Regel nicht beim Aufbau eines solchen Gerätes vorgenommen werden, zudem die Einstellungen auch bei verschiedenen Benutzern unterschiedlich gestaltet sein können.

US 2017/124271 A1 beschreibt ein System und ein Verfahren zum Teilen, Verteilen, Klassifizieren und Suchen von Konfigurationselementen für medizinische Geräte. Das Verfahren kann das Empfangen einer Anforderung umfassen, die über ein Netzwerk an einen Server übertragen wird, um auf dem Server gespeicherte medizinische Konfigurationselemente zu durchsuchen. Jedes der medizinischen Konfigurationselemente kann einer Bewertung zugeordnet sein. Jedes der medizinischen Konfigurationselemente kann angewendet werden, um eine medizinische Aufgabe auszuführen.

EP 1 091 522 A2 lehrt ein Verfahren zum automatischen Konfigurieren einer Gruppe von Geräten durch Erfassen von Konfigurationseinstellungen von einem Gerät und Anwenden einiger oder aller dieser Einstellungen auf ein anderes Gerät oder eine Gruppe von Geräten. Der Benutzer wählt ein zuvor konfiguriertes Quellgerät aus und erfasst und speichert vorzugsweise einige oder alle der Konfigurationseinstellungen elektronisch von dem Quellgerät. Diese Konfigurationseinstellungen werden dann automatisch auf ein anderes Gerät oder eine Gruppe von Zielgeräten angewendet.

US 2013/198569 offenbart ein Verfahren zum Einbetten von Konfigurationsdateien in ein von einem System erzeugten Dokument, wobei die Konfigurationsdatei Einstellungen enthält, die der Erzeugung des Dokuments zugeordnet sind.

US 2006/242148 beschreibt ein System und ein Verfahren zum Integrieren elektronischer Patienteninformationen in eine elektronische medizinische Bilddatei.

Befinden sich in einer Einrichtung mehrere medizintechnische Geräte, muss ein Benutzer in der Regel für jedes einzelne Gerät die oben genannten Eingaben vornehmen, wenn er beispielsweise eine von ihm bevorzugte Einstellung erreichen möchte, beispielsweise einfach nur sein Zugangspasswort ändern will.

Nachteil des Standes der Technik ist, dass solche Eingaben zeitaufwändig sind und sich daher negativ auf die Produktivität auswirken. Des Weiteren steigt die Gefahr von Fehlern, insbesondere, wenn gleichartige Eingaben manuell bei mehreren gleichen oder ähnlichen Geräten durchgeführt werden müssen, was sich ebenfalls negativ auf die Produktivität auswirkt. Hinzu kommt, dass gleichförmige manuelle Eingaben oftmals bei einem Benutzer eine Abneigungshaltung hervorrufen, die sich negativ auf die Arbeitsmoral auswirken kann.

Es ist eine Aufgabe der vorliegenden Erfindung, ein alternatives, komfortableres Verfahren und ein entsprechendes Gerätesystem bzw. entsprechende medizintechnische Geräte zum Management der Funktionsausübung der medizintechnischen Geräte zur Verfügung zu stellen, mit denen die oben beschriebenen Nachteile vermieden werden.

Diese Aufgabe wird durch ein Verfahren gemäß Patentanspruch 1, ein medizintechnisches Gerät gemäß Anspruch 8, ein Computerprogrammprodukt gemäß Anspruch 11 sowie durch ein computerlesbares Medium gemäß Anspruch 12 gelöst.

Gemäß der Erfindung erfolgen zum Management der Funktionsausübung der medizintechnischen Geräte bei einer Änderung oder bei einer Hinzufügung von Daten durch einen Benutzer, also einer Eingabe von benutzerspezifischen Informationen, z. B. einem Passwort, GUI-Einstellungen (GUI: graphical userinterface, dt.: Graphische Benutzerschnittstelle), Messeinstellungen oder zu bearbeitende Daten, für ein erstes der medizintechnischen Geräte in der Gerätegruppe mehrere Verfahrensschritte, die im Folgenden genauer beschrieben werden. Das erste Gerät kann dabei ein beliebiges Gerät der Gerätegruppe sein.

Zunächst erfolgt durch das erste Gerät eine automatische Erstellung eines Austauschdatensatzes, welcher zumindest einen Teil der eingegebenen benutzerspezifischen Informationen enthält. Dieses erste Gerät, an dem die Eingabe erfolgte, kann auch als "Eingabegerät" bezeichnet werden. Der Austauschdatensatz kann beispielsweise ein Passwort enthalten, welches der Benutzer im Rahmen einer Passwortänderung eingegeben hat, oder Änderungen zu Einstellungsdaten zum GUI. Die benutzerspezifischen Daten können aber auch Bilddaten sein, die von einem anderen Gerät bearbeitet, beispielsweise entrauscht, werden sollen. Wenn der Austauschdatensatz keine Daten enthält, die von einem anderen Gerät bearbeitet werden sollen, sondern insbesondere ausschließlich Daten, die der Anpassung von Geräten dienen, kann der Austauschdatensatz auch als "Anpassungsdatensatz" bezeichnet werden.

Nach der automatischen Erstellung des Austauschdatensatzes wird dieser durch das erste Gerät, welches in der Regel das Eingabegerät ist, für mindestens ein zweites Gerät der Gerätegruppe bereitgestellt. Diese Bereitstellung kann - wie später noch erläutert wird - je nach Art des Netzwerks, welches durch die Geräte der Gerätegruppe gebildet wird, an alle Geräte gleichzeitig erfolgen. Der Austauschdatensatz kann aber auch sukzessive von Gerät zu Gerät weitergeleitet werden, indem ein Gerät, welches den Austauschdatensatz empfängt, diesem mindestens einem weiteren Gerät zur Verfügung stellt. Es kann aber auch eine Mischung aus einer allgemeinen Bereitstellung und einer sukzessiven Weiterleitung erfolgen, wobei der Austauschdatensatz für eine Teilgruppe von Geräten gleichzeitig zur Verfügung gestellt wird, die diesen anschließend für andere Geräte bereitstellen, die den Austauschdatensatz bisher nicht empfangen haben.

Nach Empfang des Austauschdatensatzes durch das zweite Gerät erfolgt im Rahmen des Management der Funktionsausübung der medizintechnischen Geräte je nach Art der in dem Austauschdatensatz enthaltenen Daten eine automatische Anpassung der Einstellungen des zweiten Gerätes nach Vorgaben des Austauschdatensatzes, d. h. eine Art Abgleich der Geräte gemäß den am ersten Gerät eingegebenen benutzerspezifischen Informationen, und/oder eine Bearbeitung von Daten des Austauschdatensatzes. Das zweite Gerät steht dabei zunächst stellvertretend für beliebige weitere Geräte der Gerätegruppe, welche den Austauschdatensatz empfangen haben.

Besonders bevorzugt erfolgt die beschriebene Anpassung bei jedem Gerät, welches den Austauschdatensatz empfangen hat, oder zumindest bei demjenigen Teil der Geräte, für den die Anpassungen relevant sind, z. B. weil sie die entsprechenden Funktionen aufweisen.

Bevorzugte Beispiele für eine automatische Anpassung von Einstellungen eines Geräts sind, wie zum Teil schon erwähnt, die Änderung von Benutzerkonten wie z. B. Passwörter, Benutzernamen oder sonstigen Benutzeranmeldeinformationen, die Hinzufügung oder Änderung von Passwörtern für spezielle Anwendungen wie z. B. einen Organ-Programmeditor, für eine Qualitätssicherung oder eine Detektorkalibrierung, eine Änderung von Einstellungen der DICOM-Knoten, wie z.B. Informationen für Verbindungen zu PACS, Druckern, RIS oder entsprechenden Geräten, die Anpassung der grafischen Benutzeroberfläche oder die Kalibrierung von mobilen (Flach-)Detektoren.

Das erfindungsgemäße Verfahren zum Management der Funktionsausübung bzw. zum Abgleich von mindestens zwei, vorzugsweise jedoch noch mehr, medizintechnischen Geräten einer Gerätegruppe ist für eine große Bandbreite von medizintechnischen Geräten geeignet. Als medizintechnische Geräte werden daher hier alle Geräte verstanden, die dazu ausgelegt sind, medizintechnische (Mess-)Daten aufzunehmen, zu sammeln oder zu verarbeiten. Als medizintechnische Geräte im Sinne der Erfindung werden z. B. medizintechnische Geräte zur Messung oder Bildgebung verstanden, beispielsweise Computertomographen, Röntgengeräte oder mobile medizintechnische Detektoren. Als medizintechnische Geräte im Sinne der Erfindung werden aber auch Geräte aus der Medizintechnik verstanden, die nicht direkt Messungen an einem Patienten durchführen bzw. bildgebende Systemen bilden, sondern anderweitige Arbeit verrichten, wie z. B. Radiologieinformationssysteme (RIS), Bildarchivierungs- und Kommunikationssysteme (engl. Picture Archiving and Communication System; "PACS") oder im weitesten Sinne auch Drucker zur Ausgabe medizintechnischer Bilder oder Informationen.

Ein erfindungsgemäßes medizintechnisches Gerät ist mit einer Datenschnittstellenanordnung zur Kommunikation mit mindestens einem weiteren medizintechnischen Gerät ausgestattet. Eine solche Datenschnittstellenanordnung umfasst mindestens eine Datenschnittstelle, wobei in dem Fall, dass die Datenschnittstelle für eine bidirektionale Kommunikation ausgelegt ist, bereits eine einzige Schnittstelle ausreicht, auch wenn noch weitere Schnittstellen vorhanden sein können. Bei unidirektionalen Schnittstellen sollten mindestens zwei Schnittstellen vorhanden sein, von denen eine dazu ausgelegt ist, Daten zu empfangen, und die andere, Daten zu senden.

Das erfindungsgemäße medizintechnische Gerät umfasst des Weiteren eine, beide oder alle drei der nachfolgend beschriebenen Einheiten, die als Hard- und/oder Softwaremodule vorliegen können.

Die eine Einheit ist eine Austauschdatensatz-Erstellungseinheit, die bei Eingabe von benutzerspezifischen Informationen in dieses Gerät automatisch den Austauschdatensatz auf Basis zumindest eines Teils der eingegebenen benutzerspezifischen Informationen erstellt. Nach der Erstellung des Austauschdatensatzes stellt die Austauschdatensatz-Erstellungseinheit diesen zum Senden über eine Datenschnittstelle der Datenschnittstellenanordnung bereit.

Die zweite Einheit ist eine Anpassungseinheit, die bei Empfang eines Austauschdatensatzes von einer Datenschnittstelle der Datenschnittstellenanordnung eine automatische Anpassung der Einstellungen des medizintechnischen Geräts gemäß dem Austauschdatensatz vornimmt.

Die dritte Einheit ist eine Bearbeitungseinheit, die bei Empfang eines Austauschdatensatzes von einer Datenschnittstelle der Datenschnittstellenanordnung eine automatische Bearbeitung des Austauschdatensatzes vornimmt, z. B. Berechnungen zur Bildgebung durchführt.

Bevorzugt ist das medizintechnische Gerät zusätzlich dazu ausgelegt, einen empfangenen Austauschdatensatz zusätzlich an mindestens ein weiteres medizintechnisches Gerät weiterzuleiten.

Ein solcherart gestaltetes medizintechnisches Gerät ist besonders gut zur Einbindung in ein erfindungsgemäßes Verfahren geeignet. Im Folgenden wird ein medizintechnisches Gerät auch einfach als "Gerät" bezeichnet.

Ein erfindungsgemäßes Gerätesystem umfasst zumindest eine Gerätegruppe mit einer Mehrzahl der vorangehend beschriebenen erfindungsgemäßen medizintechnischen Geräte. Auch wenn der Begriff "Mehrzahl" die Möglichkeit umfasst, dass nur zwei Geräte in der Gerätegruppe vorhanden sind, ist es bevorzugt, dass mehr als zwei Geräte, sogar eine Vielzahl von Geräten, in dieser Gerätegruppe sind. Bevorzugt ist ferner ein Gerätesystem, wobei zumindest ein Teil der Geräte der Gerätegruppe von demselben Gerätetyp ist. Dies heißt, dass möglicherweise nur ein Computertomograph vorhanden sein könnte, aber mehrere Röntgengeräte, von denen möglicherweise wiederum eine Vielzahl von mobilen Röntgeneinheiten in der Gerätegruppe vorhanden sein kann.

Jedes medizintechnische Gerät der Gerätegruppe hat dabei Zugriff auf Geräteinformationen über mindestens ein weiteres medizintechnisches Gerät der Gerätegruppe des Gerätesystems, wobei die betreffenden Geräteinformationen eines jeden medizintechnischen Geräts der Gerätegruppe mindestens einem anderen medizintechnischen Gerät der Gerätegruppe bekannt sind. Somit "kennt" auf der einen Seite jedes Gerät mindestens ein anderes Gerät der Gruppe, und es ist auf der anderen Seite auch jedes Gerät mindestens einem anderen Gerät "bekannt".

Das Gerätesystem kann dabei nur Geräte dieser Gerätegruppe umfassen, es kann aber auch noch zusätzliche Geräte umfassen, deren Geräteinformationen den Geräten der Gerätegruppe nicht unbedingt bekannt sein müssen. Diese Geräte stehen dann jedoch außerhalb besagter Gerätegruppe der betreffenden Geräte.

Bevorzugt ist diesbezüglich entsprechend ein Verfahren, bei dem jedes Gerät der Gerätegruppe Geräteinformationen über mindestens ein weiteres Gerät der Gerätegruppe besitzt, z. B. zur Identifizierung des betreffenden Geräts und/oder über die Einstellmöglichkeiten des jeweiligen Geräts, wobei die betreffenden Geräteinformationen eines jeden Geräts mindestens einem anderen Gerät der Gerätegruppe bekannt sind.

Die zuvor genannten Komponenten des Systems oder der für die Durchführung des Verfahrens relevanten Komponenten können wie erwähnt ganz oder teilweise in Form von Softwaremodulen in einem Prozessor einer entsprechenden Steuereinrichtung eines medizintechnischen Geräts realisiert werden. Eine weitgehend softwaremäßige Realisierung hat den Vorteil, dass auch schon bisher verwendete Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Steuereinrichtung eines medizintechnischen Geräts ladbar ist, mit Programmabschnitten, um alle Schritte des erfindungsgemäßen Verfahrens auszuführen, wenn das Programm in der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z. B. Hardware-Schlüssel (Dongles etc.) zur Nutzung der Software, umfassen.

Zum Transport zur Steuereinrichtung und/oder zur Speicherung an oder in der Steuereinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuereinrichtung einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung. Dabei kann eine erfindungsgemäße Vorrichtung auch analog zu den abhängigen Verfahrensansprüchen oder Beschreibungsteilen weitergebildet sein. Insbesondere können auch einzelne Merkmale verschiedener Ausführungsbeispiele bzw. Varianten zu neuen Ausführungsbeispielen bzw. Varianten kombiniert werden.

Bevorzugt ist ein Verfahren, bei dem der Austauschdatensatz nach dem Peer-to-Peer-Prinzip (direkt) von dem ersten Gerät zu dem zweiten Gerät bzw. von einem Gerät zu einem weiteren Gerät gesendet wird. Damit erfolgt die Kommunikation nicht nach einem Client-Server-Modell. Das Netzwerk wird also durch die einzelnen Geräte aufgespannt und nicht primär von einem Server verwaltet. Auch wenn bei der Kommunikation in Form einer zentralen Peer-to-Peer-Verbindung durchaus ein Server involviert sein kann, der z. B. als Proxy- oder HUB-Server agiert, ist eine reine Peer-to-Peer-Verbindung bevorzugt.

Diesbezüglich ist das erfindungsgemäße medizintechnische Gerät vorzugsweise dazu ausgelegt, eine Peer-to-Peer-Verbindung mit anderen Geräten herzustellen. Dementsprechend ist auch ein erfindungsgemäßes Gerätesystem so gestaltet, dass der Austauschdatensatz durch ein Gerät an ein weiteres Gerät nach dem Peer-to-Peer-Prinzip von dem einen Gerät zu dem weiteren Gerät gesendet wird.

Bei einem Verfahren, bei dem der Austauschdatensatz, der von einem Gerät zu einem anderen Gerät einer Gerätegruppe gesendet wird, ist dabei bevorzugt, dass der Teil der eingegebenen benutzerspezifischen Information, den dieser Austauschdatensatz umfasst, von denjenigen Geräteinformationen abhängt, die das eine (sendende) Gerät über das zweite (empfangende) Gerät hat. Damit kann der Datenverkehr reduziert bzw. den Geräten ein speziell auf sie zugeschnittener Datensatz zugesandt werden.

Wenn beispielsweise ein Benutzer eines Computertomographen sein Passwort zusammen mit persönlichen Einstellungen der GUI ändert, die zum Teil spezifisch für das CT, aber zum Teil für die Bildgebung auch von allgemeiner Natur sind, dann könnte das CT die Passwortänderung an alle Geräte der Gerätegruppe senden, die spezifischen CT-Einstellungen nicht senden, sofern kein anderes CT in der Gerätegruppe vorhanden ist, und die Änderungen zur Bildgebung an weitere bildgebende Geräte der Gerätegruppe senden.

Bevorzugt ist des Weiteren ein Verfahren, bei dem der Austauschdatensatz an mehrere, vorzugsweise alle, Geräte der Gerätegruppe direkt gesendet wird. Das Eingabegerät informiert dabei die übrigen Geräte zur gleichen Zeit, z. B. im Rahmen der oben beschriebenen Peer-to-Peer-Verbindung.

Erfindungsgemäß sendet ein zweites Gerät, welches den Austauschdatensatz erhält, diesen an ein drittes Gerät weiter. Dies ist vorteilhaft, wenn aufgrund der Netzwerkarchitektur ein Gerät nicht direkt von einem Eingabegerät kontaktiert werden kann. Hierzu ist das dritte Gerät insbesondere eines, dessen Gerätedaten dem zweiten Gerät vorliegen. Ein medizintechnisches Gerät ist hierzu dazu ausgelegt, einen empfangenen Austauschdatensatz an mindestens ein weiteres medizintechnisches Gerät weiterzuleiten.

Wie bereits gesagt, werden Daten bevorzugt über ein Netzwerk versendet. Ein solches Netzwerk kann beispielsweise ein LAN und/oder ein WLAN umfassen. Es kann als Datenbus oder Daisy chain oder auch in anderer Weise aufgebaut sein.

Bevorzugt ist auch ein Verfahren, bei dem der Austauschdatensatz von mindestens einem zweiten Gerät vom ersten Gerät abgerufen werden kann. Der Datensatz wird also nicht (nur) bei der Eingabe, sondern auch später auf Nachfrage versendet. Dies ist insbesondere für Geräte von Vorteil, die nicht ständig angeschaltet oder nicht ständig mit dem betreffenden Netzwerk verbunden sind. Auf diese Weise können sie sich nach dem Anschalten bzw. nach dem Einklinken in das Netzwerk automatisch über die neuesten Änderungen informieren. Diese Ausführungsform kann eine Alternative, aber auch eine Ergänzung zu einer aktiven Sendung der Daten durch das erste Gerät darstellen. Erfindungsgemäß ist, dass ein Austauschdatensatz direkt nach der Erstellung versendet wird, aber auch zu einem späteren Zeitpunkt auf Anfrage noch abgerufen werden kann.

Bevorzugt ist ein Verfahren, bei dem der Austauschdatensatz zumindest Teil eines Datensatzes ist, der eine Form gemäß einem im medizintechnischen Bereich geltenden Standardprotokoll hat. Dies hat den Vorteil, dass Funktionen und Protokolle verwendet werden können, die bereits in den Steuersystemen der Geräte implementiert und für medizintechnische Zwecke ausreichend getestet sind. Bevorzugt ist dabei in erster Linie die Verwendung des DICOM-Standards (DICOM: Digital Imaging and Communications in Medicine; Deutsch: Digitale Bildgebung und -kommunikation in der Medizin). Jedoch ist je nach Anwendung auch eine Verwendung eines anderen Standards bevorzugt, insbesondere des HL7-Standards (Health Level 7, internationaler Standard für den Austausch von Daten zwischen Computersystemen im Gesundheitswesen) und/oder des IHE PDI-Standards ("Portable Document Imaging"; Empfehlungen zum Austausch von portablen optischen Medien mit Patientenbilddaten konform zum DICOM-Standard).

Dazu ist eine Datenschnittstelle eines bevorzugten medizintechnischen Geräts als Schnittstelle zum Senden und/oder Empfangen eines Datensatzes gemäß einem medizintechnischen Standardprotokoll ausgelegt, insbesondere einem Protokoll gemäß dem DICOM-Standard und/oder dem HL7-Standard und/oder dem IHE PDI-Standard. Das medizintechnische Gerät ist dabei vorteilhaft so gestaltet, dass es den Austauschdatensatz mittels einer Datenschnittstelle im Rahmen der Bereitstellung senden kann, bevorzugt nach Erstellung des Austauschdatensatzes und/oder nach Empfang einer Anfrage durch eine Datenschnittstelle des medizintechnischen Geräts.

Bevorzugt ist auch ein Verfahren, bei dem der Austauschdatensatz als Teil von digitalen Bilddaten, Teil von Metadaten, Teil von Abrechnungsdaten, Teil von Daten zu medizintechnischen Dokumenten oder Befunden oder Teil von Zusatzinformationen, wie z. B. Segmentierungen, Oberflächendefinitionen oder Bildregistrierungen, übertragen wird. Dazu ist die Austauschdatensatz-Erstellungseinheit bevorzugt dazu ausgelegt, einen Austauschdatensatz zu erzeugen, der Teil von digitalen Bilddaten, Teil von textuellen Daten, Teil von Metadaten, Teil von Abrechnungsdaten, Teil von Daten zu medizintechnischen Dokumenten oder Befunden oder Teil von Zusatzinformationen ist.

Beispielsweise könnte der Standard erfordern, dass ein Header einer bestimmten oder definierbaren Größe vorhanden sein muss, der von einem Datensatz einer vorher oder im Header festgelegten Größe gefolgt wird. Im DICOM-Standard ist ein solcher Header oftmals 795 Bytes lang (kann aber auch eine andere Anzahl von Bytes enthalten), von denen die ersten 128 Bytes in der Regel unbesetzt sind und von den Zeichen ,D', ,I', ,C' und ,M' gefolgt werden. Danach folgen im Header Informationen zu den im Datensatz enthaltenen Bildern.

Der Austauschdatensatz kann so gestaltet sein, dass nach einem Marker, z. B. den Zeichen 'DICM', in einem Header eine bestimmte Information erfolgt, mittels derer den übrigen Geräten mitgeteilt wird, dass es sich im Folgenden um einen erfindungsgemäßen Austauschdatensatz und nicht um ein normales Bild handelt.

Nun kann im folgenden Teil des Headers, der unter Umständen auch als "Metadatenteil" bezeichnet werden könnte, und der textuelle Daten aufweist, ein Informationspaket folgen, welches den betreffenden Teil der benutzerspezifischen Informationen umfasst. In diesem Fall wären die folgenden Bilddaten im Grunde unerheblich und müssten nicht unbedingt sinnvolle Informationen aufweisen.

Alternativ oder ergänzend kann im folgenden Teil des Headers aber auch eine Information folgen, dass der betreffende Teil der benutzerspezifischen Informationen in den Bilddaten zu finden ist, und um welche Art von benutzerspezifischer Information es sich handelt bzw. was mit diesen Daten geschehen soll. Ein solcherart formatierter Austauschdatensatz bietet sich an, wenn umfangreichere Datenpakete verschickt werden sollen, die z. B. Kalibrierungsdaten oder Daten zur Bearbeitung für andere Geräte umfassen. Auch eine besondere Verschlüsselung oder kombinierte Datenübertragung wäre auf diese Weise denkbar, z. B. in Form der "least significant bits" von normalen Bilddaten.

Diese Art einer kombinierten Datenübertragung könnte auch dazu genutzt werden, den betreffenden Teil der Benutzerspezifischen Daten zusammen mit normalen Bilddaten kombiniert mitzusenden, indem ein Bildbereich ausgesucht wird, der normalerweise keine für einen Arzt relevanten Informationen enthält, z. B. der äußerste Rand eines Bildes, und dort die benutzerspezifischen Daten bitweise im least significant Bit der Bilddaten codiert sind.

Ein Vorteil der Erfindung ist, dass, wenn ein Benutzer für sich spezifische Änderungen an einem Gerät des Systems vornimmt, alle anderen Geräte dieses Systems ebenfalls entsprechende Änderungen automatisch vornehmen können. Wenn z. B. ein Benutzer sein Passwort an einem der Geräte ändert, kann sich diese Passwortänderung automatisch auf alle Geräte auswirken, so dass keine weiteren Änderungen des Passwortes auf anderen Geräten notwendig sind. Der Benutzer kann sich bei Verwendung eines anderen Geräts dieses Systems direkt mit seinem neuen Passwort anmelden.

Gleiches gilt für den Fall, dass der Benutzer die graphische Bedienoberfläche (GUI) ändert oder gemäß seinen Wünschen anpasst. Die individuell konfigurierte Oberfläche kann danach auf allen Geräten (oder zumindest allen Geräten mit einer entsprechenden Bedienoberfläche) automatisch zur Verfügung stehen, ohne dass der Benutzer die Einstellungen an jedem einzelnen Gerät vornehmen muss. Typische Beispiele für eine individuelle Konfigurierung einer Bedienoberfläche sind eine Definition von favorisierten Werkzeugen, z. B. zur Bildbearbeitung (Zoomwerte, Fenstereinstellungen, R/L-Marker), von spezifischen Arbeitsschritten (Patientenregistrierung, Untersuchungen, Nachuntersuchungen, Archivierung), oder die Festlegung von Schriftfonts oder Farbschemata.

Ebenso ist eine einfache Kalibration von Detektoren möglich. Beispielsweise ist es möglich, einen portablen Röntgendetektor zusammen mit unterschiedlichen Geräten zu verwenden. Zwar muss jeder Detektor für eine optimale Bildqualität kalibriert werden. Mit der vorliegenden Erfindung muss der Detektor nur einmal kalibriert werden, und die unterschiedlichen Geräte erhalten automatisch diese Kalibrierungsdaten.

In einem Fall, in dem mehrere Geräte gleichen Typs, z. B. Röntgengeräte, mit unterschiedlicher Auslastung verwendet werden, kann die vorliegende Erfindung eine Verteilung der zur Bildbearbeitung notwendigen Rechenoperationen auf mehrere medizintechnische Geräte erreichen. Nicht oder wenig benutzte Geräte unterstützen dabei mit ihrer Rechenkapazität Geräte, die eine hohe Auslastung haben.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines bevorzugten erfindungsgemäßen Systems,
Figur 2 eine Darstellung einer bevorzugten Ausführungsform des erfindungsgemäßen Verfahrens,
Figur 3 eine Darstellung einer bevorzugten Ausführungsform eines erfindungsgemäßen medizintechnischen Geräts,
Figur 4 eine Darstellung einer bevorzugten Vorgehensweise zur Bildung eines den Austauschdatensatz enthaltenden Datensatzes,
Figur 5 eine Darstellung einer bevorzugten Vorgehensweise zur Änderung eines Benutzerpassworts,
Figur 6 eine Darstellung einer bevorzugten Vorgehensweise zur Änderung einer Benutzeroberfläche,
Figur 7 eine Darstellung einer bevorzugten Vorgehensweise zur Berechnung von Daten in dem Gerätesystem,
Figur 8 eine Darstellung einer bevorzugten Vorgehensweise zum Datenverkehr bei einer Berechnung von Daten in dem Gerätesystem.

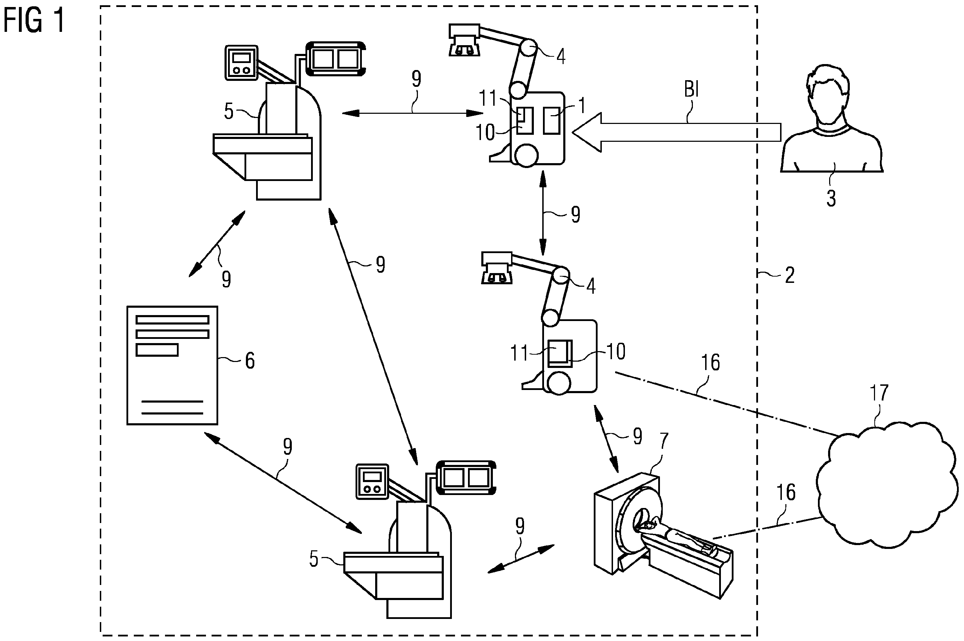

In Figur 1 ist eine Gerätegruppe 2 dargestellt, welche eine Reihe von medizintechnischen Geräten 4, 5, 6, 7 enthält, die durch logische Peer-to-Peer-Verbindungen 9 datentechnisch miteinander verbunden sind. Eine solche logische Peer-to-Peer-Verbindung 9 lässt sich durch unterschiedliche physische Verbindungen und beliebigen Netzwerkarchitekturen realisieren, z. B. mittels eines Datenbusses, an den die Geräte angeschlossen sind, durch ein sonstiges, auch drahtloses, Netzwerk oder Kombinationen verschiedener Verbindungen. Charakteristisch für die Peer-to-Peer-Verbindung 9 ist, dass sich die Geräte untereinander direkt austauschen können und Server allenfalls als eine Art Übertragungsgeräte z. B. Hub, dienen. Dies lässt sich durch entsprechende Kommunikationsprotokolle realisieren. In Figur 1 ist ein drahtloses Netzwerk symbolisiert, z. B. ein WLAN, das vorzugsweise in geeigneter Weise vor unberechtigtem Zugriff geschützt ist.

Es können auch sichere Verbindungen zu externen Diensten 17 mittels Schnittstellen 16 zu diesen externen Diensten 17 bestehen. Zum Beispiel können Daten über eine sichere Internetverbindung in eine Cloud geladen werden.

Die Geräte 4, 5, 6, 7 der Gerätegruppe 2 können gleichartig oder unterschiedlich sein. In der Figur 1 sind sowohl unterschiedliche als auch gleichartige Geräte 4, 5, 6, 7 dargestellt, beispielsweise zwei mobile Röntgengeräte 4, zwei stationäre Röntgengeräte 5, ein Bildarchivierungs- und Kommunikationssystem (PACS) 6 und ein Computertomograph 7. Die mobilen Röntgengeräte 4 sind hier durch die Rollen als mobil gekennzeichnet. Weitere mobile Geräte können auch einfache Aufnahmeeinheiten, z. B. digitale Röntgendetektoren, sein, die an mehreren anderen Geräten anbringbar sind bzw. an diesen einsetzbar sind.

Über eine Benutzerschnittstelle 1, z. B. eine Konsole 1, kann ein Benutzer 3 mit diesem mobilen Röntgengerät 4 kommunizieren und Änderungen an dessen Konfiguration vornehmen, insbesondere auch hinsichtlich benutzerspezifischer Einstellungen, wie z. B. das Passwort.

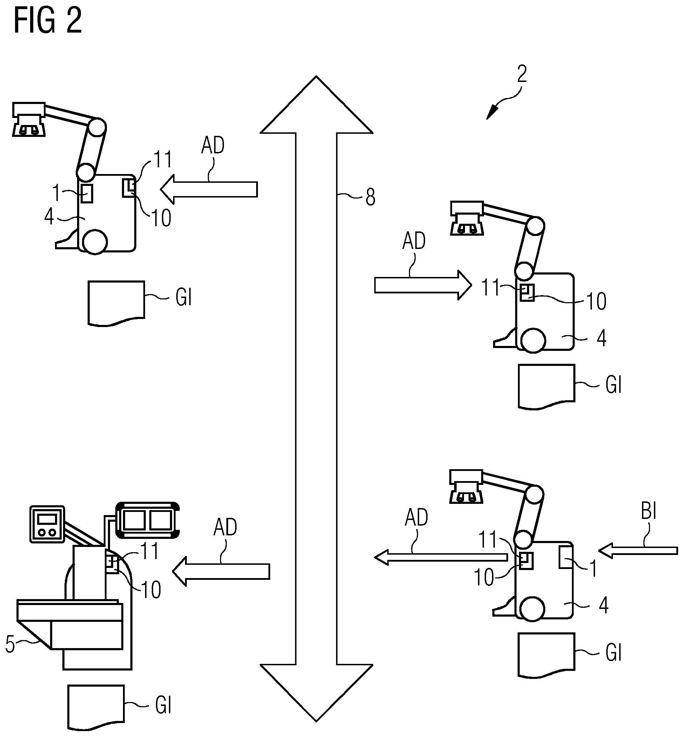

In Figur 2 wird das erfindungsgemäße Verfahren anhand eines solchen Beispiels genauer erläutert. Eine benutzerspezifische Information BI, beispielsweise eine Passwortänderung, wird in eines der mobilen Röntgengeräte 4 eingegeben, was der Pfeil rechts symbolisieren soll. Alle diese Geräte sind über Datenschnittstellenanordnungen 10 umfassend bidirektionale Schnittstellen 11 mit einem Datenbus 8 verbunden, der hier zur physischen Realisierung der in Figur 1 dargestellten Peer-to-Peer-Verbindungen 9 dient. Der Datenaustausch der medizintechnischen Geräte 4, 5 mit diesem Datenbus 8 ist mittels Pfeilen dargestellt.

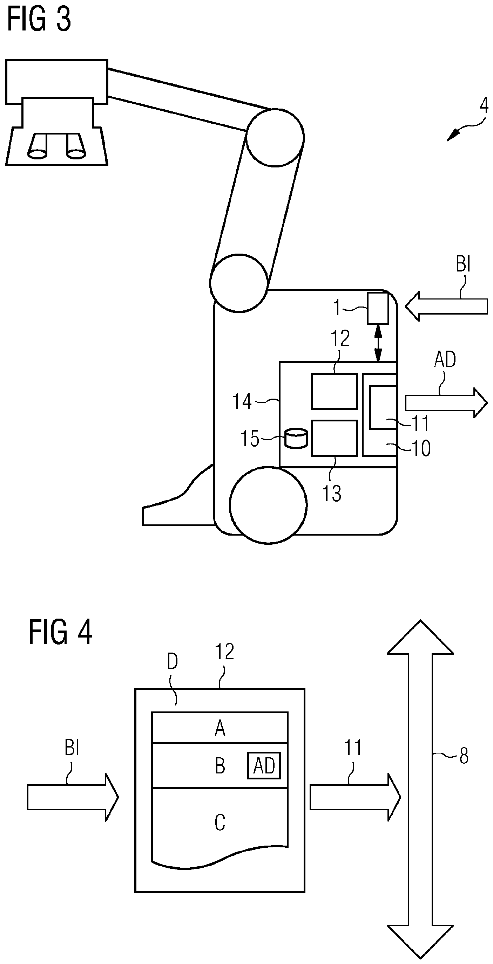

Figur 3 zeigt diesbezüglich ein bevorzugtes medizintechnisches Gerät in Form eines mobilen Röntgengeräts 4, in das gerade eine benutzerspezifische Information BI über eine Benutzerschnittstelle 1 eingegeben wird. Gezeigt ist hier auch schematisch die Datenschnittstellenanordnung 10 des Geräts 4 mit einer Schnittstelle 11 zum Austausch von Austauschdatensätzen AD mit anderen Geräten. Hier ist die Schnittstelle 11 bidirektional, so dass eine Schnittstelle 11 zum Empfang und zum Versand von Austauschdatensätzen AD ausreicht. Die Datenschnittstellenanordnung 10 kann aber auch beliebige weitere Schnittstellen umfassen. Die Schnittstellenanordnung 10 ist hier Teil einer Steuereinrichtung 14 des mobilen Röntgengeräts 4 zusammen mit einer Austauschdatensatz-Erstellungseinheit 12, einer Anpassungseinheit 13 und einer Speichereinrichtung 15. Die Schnittstellenanordnung 10 kann aber auch an anderer Stelle im Gerät 4 angeordnet und mit der Steuereinrichtung 14 verbunden sein. Insbesondere kann es sich auch um eine herkömmliche Schnittstellenanordnung 10 bzw. Schnittstelle 11 des Geräts handeln, z. B. einen Ethernet-Anschluss oder dergleichen.

Es wird an dieser Stelle auch darauf hingewiesen, dass die in den Figuren dargestellte räumliche Anordnung aller (nur durch Blöcke symbolisierte) Komponenten 1, 10, 11, 12, 12, 14, 15 in den Geräten 4, 5, 6, 7 rein willkürlich ist und daher auch unterschiedlich sein kann, selbst wenn es sich um dieselben Geräte handelt.

Bei dem in der Figur 2 dargestellten Ausführungsbeispiel erstellt die Austauschdatensatz-Erstellungseinheit 12 des in der Figur rechts unten gezeigten mobilen Röntgengeräts 4 einen Austauschdatensatz AD, der über die Schnittstelle 11 an den Datenbus 8 weitergegeben wird. Die übrigen Geräte 4 und 5 des Systems 2 erhalten die Austauschdaten AD über ihre jeweiligen Schnittstellen 11, z. B. über ein drahtloses Netzwerk. Auf diese Weise kann z. B. eine in ein Gerät eingegebene Passwortänderung an alle anderen Geräte weitergegeben werden, so dass in jedem Gerät das neue Passwort verfügbar ist.

Ein von einem Gerät empfangener Austauschdatensatz AD, der Einstellungsänderungen enthält, kann von der Anpassungseinheit 13 verwendet werden, Einstellungen in dem Gerät zu ändern, z. B. besagte Passworteinstellungen.

Unter den einzelnen Geräten ist angedeutet, dass diese Geräteinformationen GI von anderen Geräten besitzen. Z. B. können diese in der in Figur 3 symbolisierten Speichereinrichtung 15 der Steuereinrichtung 14 abgespeichert sein.

Hat nun das mobile Röntgengerät beispielsweise Geräteinformationen GI über jedes andere Gerät, kann es die Form des Austauschdatensatzes AD entsprechend gestalten. Unter der Annahme, es wären des Weiteren lediglich feststehende Röntgengeräte 5 in dem System 2 enthalten, müssten beispielsweise sämtliche benutzerspezifischen Informationen BI, die die Mobilität des mobilen Röntgengeräts 4 betreffen, nicht im Austauschdatensatz AD enthalten sein.

Würde man das vorangehende Beispiel dermaßen abwandeln, dass das mobile Röntgengerät 4 eine reine Aufnahmeeinheit wäre und die übrigen Geräte dieses mobile Röntgengerät 4 nutzen bzw. bildbearbeitende Geräte für dieses mobile Röntgengerät 4 sind, so würde sich anbieten, dass der Austauschdatensatz AD Kalibrierungsdaten des mobilen Röntgengerätes 4 enthält, welche nun jedes der anderen Geräte bei der nächsten Verwendung des mobilen Röntgengeräts 4 an dem jeweiligen Gerät nutzen könnte.

In Figur 4 ist die Bildung eines bevorzugten Datensatzes D mit einem Austauschdatensatz AD dargestellt. Die eingegebenen benutzerspezifischen Informationen BI werden durch die Austauschdatensatz-Erstellungseinheit 12 in einen Austauschdatensatz AD konvertiert, der in einen Datensatz D gemäß des DICOM-Standards integriert wird. Dazu wird der Datensatz D so gestaltet, dass er einen Header A, einen Teil mit textuellen Daten B und einen Teil mit visuellen Daten C umfasst, wobei die visuellen Daten C den DICOM-Bilddaten entsprechen, aber nicht zwingend sinnvolle Bilder sein müssen. Derjenige Teil der benutzerspezifischen Informationen BI, welcher an die anderen Geräte weitergesendet werden soll, wird in einen Datensatz AD geschrieben, der bevorzugt je nach dem Zweck des Austauschs in Teil B oder C abgelegt wird. In der vorliegenden Darstellung ist der Austauschdatensatz AD in Teil B enthalten.

In dem Header A sollten Informationen stehen, die den übrigen Geräten mitteilen, dass es sich bei diesem Datenpaket um einen Datensatz D umfassend einen Austauschdatensatz AD gemäß der Erfindung handelt, wo die übertragenen benutzerspezifischen Informationen BI zu finden sind, und ggf., mit welchem Schlüssel sie verschlüsselt worden sind.

Der fertige Austauschdatensatz AD wird, wie in Figur 2 gezeigt, mittels der Schnittstelle 11 an die anderen Geräte gesendet, beispielsweise durch den hier dargestellten Datenbus 8.

Wie vorangehend gesagt, kann die weitergegebene benutzerspezifische Information BI in Form des Austauschdatensatzes AD in Teil B oder Teil C enthalten sein. Beispielsweise bietet es sich an, Konfigurierungsdaten im Teil B abzuspeichern und Kalibrierungsdaten, zumindest sofern sie umfangreich sind, in Teil C.

In dem Fall, dass Bilddaten in dem Austauschdatensatz AD enthalten sind, die von einem anderen Gerät des Systems 2 bearbeitet werden sollen, sollten diese im Teil C abgespeichert sein. In Teil B können Daten abgespeichert werden, die Aufschluss darüber geben, von welchem Gerät diese Bilddaten stammen und was mit diesen Daten geschehen soll.

Die bearbeiteten Bilddaten können auf dieselbe Weise wieder zurückgesandt werden. Im Header A könnte dabei der Hinweis stehen, dass es sich hier um bearbeitete Bilddaten handelt.

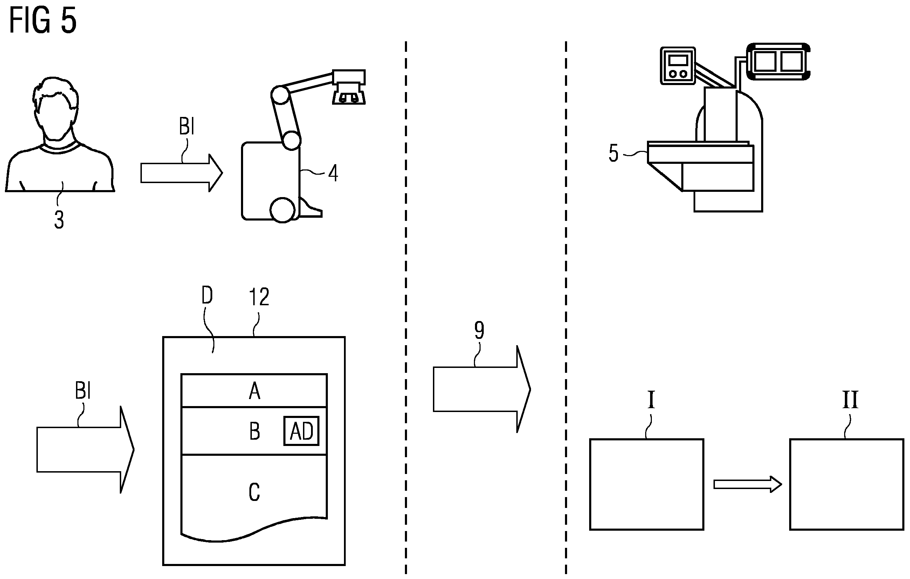

Figur 5 zeigt den Fall, dass ein Passwort geändert wird. Die Datenschnittstellen sind aus Gründen der Übersicht hier und in den folgenden Figuren nicht mehr explizit eingezeichnet.

Ändert der Benutzer 3 sein Passwort an einem der medizintechnischen Geräte, beispielsweise einem mobilen Röntgengerät 4, so wird ein Datensatz D erstellt, wie er z. B. in Figur 4 dargestellt ist. Teil A enthält eine Kennzeichnung, dass in diesem Datensatz D ein Austauschdatensatz AD zu finden ist, und ggf. zusätzliche Daten zu dem Format oder dem Ort dieses Austauschdatensatzes AD.

Teil B enthält den Austauschdatensatz AD mit verschlüsselten Informationen zum Benutzer 3, der Funktion des Benutzers 3 und dem geänderten Passwort.

Über eine Peer-to-Peer-Verbindung 9 wird der Datensatz D zu einem anderen medizintechnischen Gerät 5, beispielsweise einem stationären Röntgengerät 5, gesandt. Dort wird der Datensatz D bzw. der Austauschdatensatz AD in einem ersten Schritt I decodiert und in einem zweiten Schritt II das lokale Benutzermanagement des stationären Röntgengeräts 5 entsprechend angepasst, indem das Benutzerpasswort auch in dem stationären Röntgengerät 5 geändert wird.

Der Teil C wird in diesem Beispielfall nicht für Informationen benötigt.

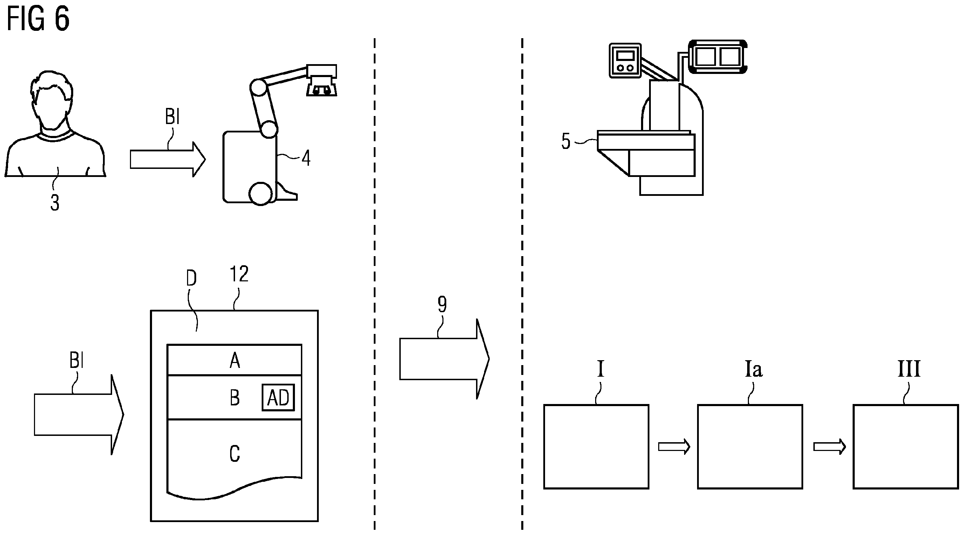

Figur 6 zeigt den Fall, dass die Benutzeroberfläche geändert wird. Ändert der Benutzer 3 die Einstellungen zu seiner persönlichen Benutzeroberfläche an einem der medizintechnischen Geräte, beispielsweise wieder einem mobilen Röntgengerät 4, so wird ein Datensatz D erstellt, wie er ebenfalls in Figur 4 dargestellt ist. Teil A enthält auch hier eine Kennzeichnung, dass in diesem Datensatz D ein Austauschdatensatz AD zu finden ist, und ggf. zusätzliche Daten zu dem Format oder dem Ort dieses Austauschdatensatzes AD. Zusätzlich enthält Teil A Informationen zum Systemtyp, so dass andere Systeme entscheiden können, ob die Änderungen zur Benutzeroberfläche für sie relevant sind.

Teil B enthält den Austauschdatensatz mit verschlüsselten Informationen zum Benutzer 3, der Funktion des Benutzers 3 und den Änderungen, z. B. zu Farben, favorisierten Werkzeugen und/oder Spracheinstellungen.

Über eine Peer-to-Peer Verbindung 9 wird der Datensatz D wie auch bereits in Figur 5 zu einem anderen medizintechnischen Gerät 5 gesandt. Dort wird der Datensatz D bzw. der Austauschdatensatz AD in einem ersten Schritt I decodiert, wobei im Unterschied zu der voran beschriebenen Passwortänderung in einem Schritt Ia ein Systemcheck erfolgt, bei dem überprüft wird, ob das empfangende Gerät 5 einen betreffend die Änderungen kompatiblen Systemtyp hat (bzw. einem solchen Systemtyp entspricht) und ob der Benutzer 3 die erforderlichen Rechte auf diesem System hat.

War der Systemcheck erfolgreich, erfolgt in einem weiteren Schritt III eine Anpassung der lokalen Einstellungen des stationären Röntgengeräts 5.

Der Teil C wird auch in dem Beispielfall nicht für Informationen benötigt.

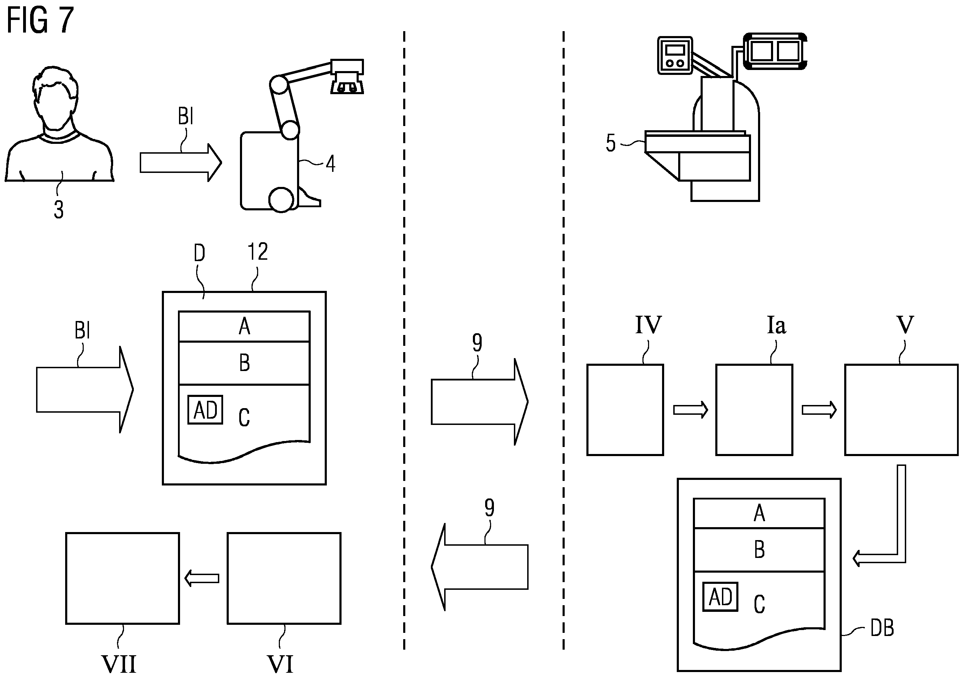

Figur 7 zeigt den Fall, dass Daten, die von einem medizintechnischen Gerät, hier wieder dem mobilen Röntgengerät 4, aufgenommen worden sind, von einem anderen Gerät, dem stationären Röntgengerät 5, rekonstruiert werden.

Teil A enthält hier eine Kennzeichnung, dass in diesem Datensatz D ein Austauschdatensatz AD zu finden ist, zu dem Berechnungen durchgeführt werden sollen, und ggf. zusätzliche Daten zu dem Format oder dem Ort dieses Austauschdatensatzes AD.

Teil B enthält Informationen zur gewünschten Berechnung.

Teil C enthält den Austauschdatensatz AD mit den (verschlüsselten) Rohdaten.

Über eine Peer-to-Peer-Verbindung 9 wird der Datensatz D zu dem stationären Röntgengerät 5 gesandt. Dort wird der Datensatz D bzw. der Austauschdatensatz AD in einem ersten Schritt IV ggf. decodiert und die Rohdaten für das empfangende System bereitgestellt, wobei in einem Schritt Ia wieder ein Systemcheck erfolgt, bei dem überprüft wird, ob das empfangende Gerät 5 einen kompatiblen Systemtyp hat (bzw. einem solchen Systemtyp entspricht) und ob der Benutzer die erforderlichen Rechte auf diesem System hat. War der Systemcheck erfolgreich, erfolgt in einem weiteren Schritt V die gewünschte Berechnung.

Im Unterschied zu den beiden vorangehenden Figuren erfolgt nun eine Antwort in Form der berechneten Bilddaten. Von dem stationären Röntgengerät 5 wird ein zweiter Datensatz DB mit Austauschdaten erstellt. Dieser enthält in Teil A einen Hinweis, dass es sich um berechnete Daten handelt, und ggf. einen Hinweis auf die Rohdaten, damit bei vielen entsprechenden Vorgängen eine Zuordnung einfacher ist. Teil B enthält Informationen zu den berechneten Bilddaten und Teil C diese berechneten Bilddaten.

Dieser Datensatz DB wird über eine Peer-to-Peer-Verbindung 9 zurück zu dem mobilen Röntgengerät 4 gesandt. Dort wird der Datensatz DB in einem Schritt VI empfangen und ggf. aufbereitet und in einem folgenden Schritt VII in dem lokalen Speicher des mobilen Röntgengerätes abgespeichert oder an ein PACS weitergesendet. Der Datensatz DB kann zusätzlich oder alternativ direkt an ein PACS gesendet werden.

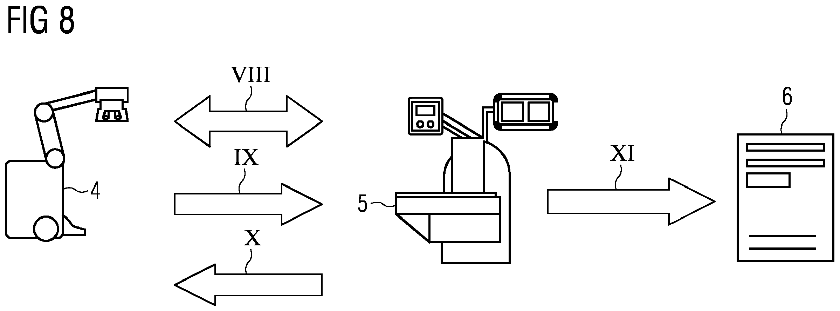

Diese Weitersendung wird in Figur 8 dargestellt. In dieser Darstellung wird die Kommunikation bzw. der Datenverkehr verdeutlicht. Das mobile Röntgengerät 4 und das stationäre Röntgengerät 5 kommunizieren zunächst in Schritt VIII, ob eine Berechnung von Daten des mobilen Röntgengeräts 4 auf dem stationären Röntgengerät 5 möglich ist. Danach erfolgt, wie oben beschrieben, in Schritt IX die Übersendung der Rohdaten. Nach der Berechnung sendet das stationäre Röntgengerät 5 die berechneten Daten in Schritt X zurück an das mobile Röntgengerät 4 und in Schritt XI an ein PACS 6. Die Schritte X und XI können nacheinander oder gleichzeitig erfolgen. Es ist zum Beispiel möglich, dass das stationäre Röntgengerät 5 die berechneten Daten an das Netzwerk sendet, wo sie theoretisch von allen Geräten empfangen werden könnten, die Daten jedoch nur von dem mobilen Röntgengerät 4 und dem PACS 6 angenommen und von den anderen Geräten ignoriert oder verworfen werden.

Es wird abschließend noch einmal darauf hingewiesen, dass es sich bei den vorhergehend detailliert beschriebenen Verfahren sowie bei dem dargestellten System lediglich um Ausführungsbeispiele handelt. Beispielsweise kann auch der gesamte Datensatz D, DB, welcher den Austauschdatensatz enthält, wieder selbst als ein Austauschdatensatz betrachtet werden. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

### Bezugszeichenliste

- 1: Benutzerschnittstelle / Konsole
- 2: Gerätegruppe
- 3: Benutzer
- 4: Mobiles Röntgengerät
- 5: Stationäres Röntgengerät
- 6: PACS
- 7: Computertomograph
- 8: Datenbus
- 9: Peer-to-Peer Verbindungen
- 10: Datenschnittstellenanordnung
- 11: Schnittstelle
- 12: Austauschdatensatz-Erstellungseinheit
- 13: Anpassungseinheit
- 14: Steuereinrichtung
- 15: Speichereinrichtung
- 16: Interface zu externen Diensten
- 17: Externer Dienst
- A: Header
- AD: Austauschdatensatz
- B: Metadaten
- BI: Benutzerspezifische Information
- C: Weitere Daten
- D,: DB Datensatz

## Patentansprüche

1. Verfahren zum Management der Funktionsausübung von medizintechnischen Geräten (4, 5, 6, 7) einer Gerätegruppe (2) bei Eingabe von benutzerspezifischen Informationen (BI), für ein erstes der Geräte (4) in der Gerätegruppe (2), umfassend die Schritte:
- automatische Erstellung eines Austauschdatensatzes (AD), umfassend zumindest einen Teil der eingegebenen benutzerspezifischen Information (BI), mittels des ersten Geräts (4),
- Bereitstellen dieses Austauschdatensatzes (AD) durch das Eingabegerät (4) für mindestens ein zweites Gerät (4, 5, 6, 7) der Gerätegruppe (2), wobei ein Austauschdatensatz direkt nach der Erstellung an das zweite Gerät (4, 5, 6, 7) versendet wird, aber auch zu einem späteren Zeitpunkt auf Anfrage noch abgerufen werden kann, und wobei das zweite Gerät (4, 5, 6, 7), welches den Austauschdatensatz (AD) erhält, diesen an ein drittes Gerät (4, 5, 6, 7) weitersendet,
- automatische Anpassung der Einstellungen des zweiten Gerätes (4, 5, 6, 7) und dritten Geräts (4, 5, 6, 7) gemäß dem Austauschdatensatz (AD) und/oder Bearbeitung von Daten des Austauschdatensatzes (AD).

2. Verfahren nach Anspruch 1, wobei der Austauschdatensatz (AD) nach dem Peer-to-Peer-Prinzip von dem ersten Gerät (4, 5, 6, 7) zu dem zweiten Gerät (4, 5, 6, 7) gesendet wird, wobei eine reine Peer-to-Peer-Verbindung (9) bevorzugt ist.

3. Verfahren nach einem der vorangehenden Ansprüche, wobei
i) jedes Gerät (4, 5, 6, 7) der Gerätegruppe (2) Geräteinformationen (GI) über mindestens ein weiteres Gerät (4, 5, 6, 7) der Gerätegruppe (2) umfasst und
ii) die betreffenden Geräteinformationen (GI) eines jeden Geräts (4, 5, 6, 7) mindestens einem anderen Gerät (4, 5, 6, 7) der Gerätegruppe (2) bekannt sind,
wobei bevorzugt der Austauschdatensatz (AD), der von einem ersten Gerät (4, 5, 6, 7) zu einem zweiten Gerät (4, 5, 6, 7) gesendet wird, nur einen Teil der eingegebenen benutzerspezifischen Information (BI) umfasst, der von den Geräteinformationen (GI) abhängt, den das erste Gerät (4, 5, 6, 7) über das zweite Gerät (4, 5, 6, 7) hat.

4. Verfahren nach einem der vorangehenden Ansprüche, wobei der Austauschdatensatz (AD) an mehrere, vorzugsweise alle, Geräte (4, 5, 6, 7) der Gerätegruppe (2) direkt gesendet wird.

5. Verfahren nach einem der vorangehenden Ansprüche, wobei der Austauschdatensatz (AD) von mindestens einem zweiten Gerät (4, 5, 6, 7) vom ersten Gerät (4) abgerufen werden kann.

6. Verfahren nach einem der vorangehenden Ansprüche, wobei der Austauschdatensatz (AD) zumindest Teil eines Datensatzes (D) ist, der eine Form gemäß einem im medizintechnischen Bereich geltenden Standardprotokoll hat, bevorzugt nach dem DICOM-Standard.

7. Verfahren nach einem der vorangehenden Ansprüche, wobei der Austauschdatensatz (AD) als Teil von digitalen Bilddaten (B), Teil von textuellen Daten, Teil von Metadaten, Teil von Abrechnungsdaten, Teil von Daten zu medizintechnischen Dokumenten oder Befunden oder Teil von Zusatzinformationen übertragen wird.

8. Medizintechnisches Gerät (4, 5, 6, 7), welches mit einer Datenschnittstellenanordnung (10) umfassend mindestens eine Datenschnittstelle (11) zur Kommunikation mit mindestens einem weiteren medizintechnischen Gerät (4, 5, 6, 7) ausgestattet ist, wobei das medizintechnische Gerät (4, 5, 6, 7)
i) eine Austauschdatensatz-Erstellungseinheit (12) umfasst, die bei Eingabe von benutzerspezifischen Informationen (BI) in dieses Gerät (4, 5, 6, 7) automatisch einen Austauschdatensatz (AD) erstellt, welcher zumindest einen Teil der eingegebenen benutzerspezifischen Information (BI) umfasst und zum Senden über die Datenschnittstellenanordnung (10) bereitstellt, und
ii) eine Anpassungs- oder Bearbeitungseinheit (13) umfasst, die bei Empfang eines Austauschdatensatzes (AD) von der Datenschnittstellenanordnung (10) eine automatische Anpassung der Einstellungen des medizintechnischen Geräts (4, 5, 6, 7) gemäß dem Austauschdatensatz (AD) vornimmt und/oder eine Bearbeitung von Daten des Austauschdatensatzes (AD) vornimmt, **dadurch gekennzeichnet, dass** das medizintechnische Gerät (4, 5, 6, 7) dazu ausgelegt ist, dass ein Austauschdatensatz (AD) direkt nach der Erstellung an ein zweites Gerät (4, 5, 6, 7) versendet wird, aber auch zu einem späteren Zeitpunkt auf Anfrage noch abgerufen werden kann und wobei das medizintechnische Gerät (4, 5, 6, 7) außerdem dazu ausgelegt ist, einen empfangenen Austauschdatensatz (AD) an ein drittes Gerät (4, 5, 6, 7) weiterzusenden.

9. Gerätesystem (2) umfassend zumindest eine Gerätegruppe (2) mit einer Mehrzahl von medizintechnischen Geräten (4, 5, 6, 7) nach Anspruch 8, wobei jedes medizintechnische Gerät (4, 5, 6, 7) einer Gerätegruppe Zugriff auf Geräteinformationen (GI) über mindestens ein weiteres medizintechnisches Gerät (4, 5, 6, 7) der Gerätegruppe hat und die betreffenden Geräteinformationen (GI) eines jeden medizintechnischen Geräts (4, 5, 6, 7) der Gerätegruppe mindestens einem anderen medizintechnischen Gerät (4, 5, 6, 7) der Gerätegruppe bekannt sind.

10. Gerätesystem nach Anspruch 9, wobei zumindest ein Teil der Geräte (4, 5, 6, 7) der Gerätegruppe (2) vom selben Gerätetyp sind.

11. Computerprogrammprodukt mit einem Computerprogramm, welches direkt in eine Speichereinrichtung (15) einer Steuereinrichtung (14) eines medizintechnischen Geräts (4, 5, 6, 7) ladbar ist, mit Programmabschnitten, um alle Schritte des Verfahrens nach einem der Ansprüche 1 bis 7 auszuführen, wenn das Computerprogramm in der Steuereinrichtung (14) des medizintechnischen Geräts (4, 5, 6, 7) ausgeführt wird.

12. Computerlesbares Medium, auf welchem von einer Rechnereinheit einlesbare und ausführbare Programmabschnitte gespeichert sind, um alle Schritte des Verfahrens nach einem der Ansprüche 1 bis 7 auszuführen, wenn die Programmabschnitte von der Rechnereinheit ausgeführt werden.

## Claims

1. Method for managing the functionality of medical devices (4, 5, 6, 7) in a device group (2) upon input of user-specific information (BI), for a first of the devices (4) in the device group (2), comprising the steps of:
- automatic creation of a replacement data set (AD), comprising at least part of the input user-specific information (BI), by means of the first device (4),
- provision of said replacement data set (AD) by the input device (4) for at least one second device (4, 5, 6, 7) in the device group (2), wherein a replacement data set is sent to the second device (4, 5, 6, 7) directly after the creation, but can also still be retrieved at a later point in time on request, and wherein the second device (4, 5, 6, 7) which contains the replacement data set (AD) forwards this to a third device (4, 5, 6, 7),
- automatic adjustment of the settings of the second device (4, 5, 6, 7) and third device (4, 5, 6, 7) according to the replacement data set (AD) and/or processing of data of the replacement data set (AD).

2. Method according to claim 1, wherein the replacement data set (AD) is sent in accordance with the peer-to-peer principle from the first device (4, 5, 6, 7) to the second device (4, 5, 6, 7), wherein a pure peer-to-peer connection (9) is preferred.

3. Method according to one of the preceding claims, wherein
i) each device (4, 5, 6, 7) in the device group (2) includes device information (GI) about at least one further device (4, 5, 6, 7) in the device group (2) and
ii) the relevant device information (GI) of each device (4, 5, 6, 7) is known to at least one other device (4, 5, 6, 7) in the device group (2),
wherein preferably the replacement data set (AD), which is sent from a first device (4, 5, 6, 7) to a second device (4, 5, 6, 7), includes only part of the input user-specific information (BI) which depends on the device information (GI) which the first device (4, 5, 6, 7) has about the second device (4, 5, 6, 7).

4. Method according to one of the preceding claims, wherein the replacement data set (AD) is sent directly to multiple, preferably all, devices (4, 5, 6, 7) in the device group (2).

5. Method according to one of the preceding claims, wherein the replacement data set (AD) can be retrieved by at least one second device (4, 5, 6, 7) from the first device (4).

6. Method according to one of the preceding claims, wherein the replacement data set (AD) is at least part of a data set (D) which has a form in accordance with a standard protocol applicable in the field of medical technology, preferably according to the DICOM standard.

7. Method according to one of the preceding claims, wherein the replacement data set (AD) is transmitted as part of digital image data (B), part of text data, part of metadata, part of accounting data, part of data on medical documents or findings or part of additional information.

8. Medical device (4, 5, 6, 7) which is fitted with a data interface arrangement (10) comprising at least one data interface (11) for communication with at least one further medical device (4, 5, 6, 7), wherein the medical device (4, 5, 6, 7)
i) comprises a replacement data set creation unit (12) that automatically creates a replacement data set (AD) upon input of user-specific information (BI) into said device (4, 5, 6, 7), which replacement data set (AD) contains at least part of the input user-specific information (BI) and provides it for sending via the data interface arrangement (10),
and
ii) comprises an adjustment or processing unit (13) which upon receipt of a replacement data set (AD) from the data interface arrangement (10) performs an automatic adjustment of the settings of the medical device (4, 5, 6, 7) in accordance with the replacement data set (AD) and/or performs editing of data of the replacement data set (AD),
**characterised in that**
the medical device (4, 5, 6, 7) is designed such that a replacement data set (AD) is sent to a second device (4, 5, 6, 7) immediately after the creation, but can also still be retrieved at a later point in time on request and wherein the medical device (4, 5, 6, 7) is also designed to forward a received replacement data set (AD) to a third device (4, 5, 6, 7) .

9. Device system (2) comprising at least one device group (2) having a plurality of medical devices (4, 5, 6, 7) according to claim 8, wherein each medical device (4, 5, 6, 7) in a device group has access to device information (GI) about at least one other medical device (4, 5, 6, 7) in the device group and the relevant device information (GI) of each medical device (4, 5, 6, 7) in the device group is known to at least one other medical device (4, 5, 6, 7) in the device group.

10. Device system according to claim 9, wherein at least some of the devices (4, 5, 6, 7) in the device group (2) are of the same device type.

11. Computer program product having a computer program, which can be loaded directly into a memory device (15) of a control device (14) of a medical device (4, 5, 6, 7), having program sections in order to execute all the steps of the method according to one of claims 1 to 7, if the computer program is executed in the control device (14) of the medical device (4, 5, 6, 7).

12. Computer-readable medium, on which are stored program sections that can be read and executed by a computer unit, in order to perform all the steps of the method according to one of claims 1 to 7, if the program sections are executed by the computer unit.

## Revendications

1. Procédé de gestion de la fonctionnalité d'appareils (4, 5, 6, 7) de la technique médicale d'un groupe (2) d'appareils lors de l'entrée d'informations (BI) spécifiques à l'utilisateur, pour un premier des appareils (4) du groupe (2) d'appareils, comprenant les stades :
- établissement automatique d'un jeu (AD) de données d'échange, comprenant au moins une partie de l'information (BI) entrée spécifique à l'utilisateur, au moyen du premier appareil (4),
- mise de ce jeu (AD) de données d'échange par l'appareil (4) d'entrée à disposition d'au moins un deuxième appareil (4, 5, 6, 7) du groupe (2) d'appareils, un jeu de données d'échange étant envoyé directement après l'établissement au deuxième appareil (4, 5, 6, 7), mais pouvant être appelé encore sur demande à un instant ultérieur, et le deuxième appareil (4, 5, 6, 7), qui reçoit le jeu (AD) de données d'échange, l'acheminant à un troisième appareil (4, 5, 6, 7),
- adaptation automatique des réglages du deuxième appareil (4, 5, 6, 7) et du troisième appareil (4, 5, 6, 7) suivant le jeu (AD) de données d'échange et/ou le traitement de données du jeu (AD) de données d'échange.

2. Procédé suivant la revendication 1, dans lequel on envoie le jeu (AD) de données d'échange suivant le principe peer-to-peer du premier appareil (4, 5, 6, 7) au deuxième appareil (4, 5, 6, 7), une liaison (9) peer-to-peer pure étant préférée.

3. Procédé suivant l'une des revendications précédentes, dans lequel
i) chaque appareil (4, 5, 6, 7) du groupe (2) d'appareils comprend des informations (GI) d'appareils sur au moins un autre appareil (4, 5, 6, 7) du groupe (2) d'appareils et
ii) les informations (GI) d'appareils concernés de chaque appareil (4, 5, 6, 7) sont connues d'au moins un autre appareil (4, 5, 6, 7) du groupe (2) d'appareils,
dans lequel, de préférence, le jeu (AD) de données d'échange, qui est envoyé d'un premier appareil (4, 5, 6, 7) à un deuxième appareil (4, 5, 6, 7), ne contient qu'une partie de l'information (BI) entrée spécifique à l'utilisateur, laquelle dépend des informations (GI ) d'appareils que le premier appareil (4, 5, 6, 7) a sur le deuxième appareil (4, 5, 6, 7).

4. Procédé suivant l'une des revendications précédentes, dans lequel on envoie le jeu (AD) de données d'échange directement à plusieurs, de préférence à tous les appareils (4, 5, 6, 7) du groupe (2) d'appareils.

5. Procédé suivant l'une des revendications précédentes, dans lequel le jeu (AD) de données d'échange peut être appelé du au moins un deuxième appareil (4, 5, 6, 7) par le premier appareil (4).

6. Procédé suivant l'une des revendications précédentes, dans lequel le jeu (AD) de données d'échange fait au moins partie d'un jeu (D) de données, qui a une forme suivant un protocole de norme valant dans le domaine médical, de préférence suivant la norme DICOM.

7. Procédé suivant l'une des revendications précédentes, dans lequel le jeu (AD) de données d'échange transmet, comme partie de données (B) d'image numériques, une partie de données de texte, une partie de métadonnées, une partie de données de comptabilité, une partie de données de documents ou de constatation de la technique médicale ou une partie d'informations supplémentaires.

8. Appareil (4, 5, 6, 7) de la technique médicale, qui est équipé d'un système (10) d'interface de données, comprenant au moins une interface (11) de données pour communiquer avec au moins un autre appareil (4, 5, 6, 7) de la technique médicale, l'appareil (4, 5, 6, 7) de la technique médicale comprenant
i) une unité (12) d'établissement d'un jeu de données d'échange, qui, lors de l'entrée d'informations (BI) spécifiques à l'utilisateur dans cet appareil (4, 5, 6, 7), établit automatiquement un jeu (AD) de données d'échange, qui comprend au moins une partie de l'information (BI) entrée spécifique à l'utilisateur et la met à disposition pour l'envoi par le système (10) d'interface de données et
ii) comprend une unité (13) d'adaptation et de traitement, qui, à la réception d'un jeu (AD) de données d'échange du système (10) d'interface de données, effectue une adaptation automatique des réglages de l'appareil (4, 5, 6, 7) de la technique médicale suivant le jeu (AD) de données d'échange et/ou effectue un traitement de données du jeu (AD) de données d'échange,
**caractérisé en ce que**
l'appareil (4, 5, 6, 7) de la technique médicale est conçu de manière à envoyer un jeu (AD) de données d'échange directement après l'établissement à un deuxième appareil (4, 5, 6, 7), mais à pouvoir aussi l'appeler encore sur demande à un instant ultérieur et dans lequel l'appareil (4, 5, 6, 7) de la technique médicale est conçu, en outre, pour acheminer un jeu (AD) de données d'échange reçues à un troisième appareil (4, 5, 6, 7).

9. Système (2) d'appareil, comprenant au moins un groupe (2) d'appareils ayant une pluralité d'appareils (4, 5, 6, 7) de la technique médicale suivant la revendication 8, chaque appareil (4, 5, 6, 7) de la technique médicale d'un groupe d'appareils ayant accès à des informations (GI) d'appareils sur au moins un autre appareil (4, 5, 6, 7) de la technique médicale du groupe d'appareils et les informations (GI) d'appareils concernés de chaque appareil (4, 5, 6, 7) de la technique médicale du groupe d'appareils étant connues au moins d'un autre appareil (4, 5, 6, 7) de la technique médicale du groupe d'appareils.

10. Système d'appareil suivant la revendication 9, dans lequel au moins une partie des appareils (4, 5, 6, 7) du groupe (2) d'appareils sont du même type d'appareil.

11. Produit de programme d'ordinateur ayant un programme d'ordinateur, qui peut être chargé directement dans un dispositif (15) de mémoire d'un dispositif (14) de commande d'un appareil (4, 5, 6, 7) de la technique médicale, comprenant des parties de programme, pour effectuer tous les stades du procédé suivant l'une des revendications 1 à 7, lorsque le programme d'ordinateur est réalisé dans le dispositif (14) de commande de l'appareil (4, 5, 6, 7) de la technique médicale.

12. Support déchiffrable par ordinateur, sur lequel sont mises en mémoire des parties de programme pouvant être déchiffrées et réalisées par une unité informatique, afin d'effectuer tous les stades du procédé suivant l'une des revendications 1 à 7, lorsque les parties de programme sont réalisées par l'unité informatique.
